# EUROPEAN PATENT SPECIFICATION

(11) **EP 1 323 715 B1**
(45) Date of publication and mention of the grant of the patent: **30.06.2004**
(21) Application number: 03006153.5
(22) Date of filing: 15.04.1998
(51) Int. Cl.: C07D 307/20, C07D 309/32, C07D 315/00

(54) **Inhibitors of cholesterol esterase**
Cholesterin Esterase Inhibitoren
Inhibiteurs de cholestérol esterase

(30) Priority: 16.04.1997 US 41988
(43) Date of publication of application: 02.07.2003
(62) Divisional of application: 98918098.9
(73) Proprietor: The University of New Mexico, Albuquerque, NM 87131 (US)
(72) Inventor: Deck, Lorraine., Albuquerque,NM 87110 (US); Vander Jagt, David.L., Albuquerque, NM 87106 (US)
(74) Representative: Klunker . Schmitt-Nilson . Hirsch

(56) References cited:
- FR-A- 2 741 620
- US-A- 3 227 736
- US-A- 5 025 017
- US-A- 5 059 696
- US-A- 5 210 228
- YAMAMATO M: "CYCLISATION OF ACETYLENE CARBOXYLIC ACID.SYNTHESIS OF METHYLENEBUTYROLACTONES" JOURNAL OF THE CHEMICAL SOCIETY, PERKIN TRANSACTIONS 1, CHEMICAL SOCIETY. LETCHWORTH, GB, 1981, pages 582-587, XP002140973 ISSN: 0300-922X
- ZUPAN L A ET AL: "STRUCTURAL DETERMINANTS OF HALOENOL LACTONE-MEDIATED SUICIDE INHIBITION OF CANINE MYOCARDIAL CALCIUM-INDEPENDENT PHOSPHOLIPASE A2" JOURNAL OF MEDICINAL CHEMISTRY, AMERICAN CHEMICAL SOCIETY, US, vol. 36, no. 1, 1993, pages 95-100, XP002140909 ISSN: 0022-2623
- DAI W ET AL: "NEW APROACH TO THE SYNTHEIS OF ALKYL-SUBSTITUTED ENOL LACTONE SYSTEMS, INHIBITORS OF THE SERINE PROTEASE ELASTASE" JOURNAL OF ORGANIC CHEMISTRY, AMERICAN CHEMICAL SOCIETY. EASTON, US, vol. 58, 1993, pages 1900-1908, XP002239892 ISSN: 0022-3263
- Boulanger et al: 'Structure-Activity Study of 6- Substituted 2-Pyranones as Inactivators of alpha -Chymotrypsin', J.MED.CHEM., vol.29, 1986, pages 1159-1163 XP000882704

## Description

### RELATED APPLICATION

The present application is based on U.S. Provisional Patent Application No. 60/041,988 filed April 16, 1997, the entire disclosure and contents of which is hereby incorporated by reference.

### BACKGROUND OF THE INVENTION

### Field of the Invention

The present invention relates to inhibitors of cholesterol esterase.

### Description of the Prior Art

Primary hypercholesterolemia is an established risk factor of atherosclerosis and coronary heart disease (CHD). Epidemiological data indicate a positive relationship between serum LDL-cholesterol and CHD which is the leading cause of death in both men and women in the United States. Clinical trials with cholesterol-lowering regimens are beneficial in the prevention of CHD morbidity and mortality. A variety of regimens have been used to lower serum cholesterol including diet restriction, nicotinic acid, bile acid sequestrants, and HMGCoA reductase inhibitors. Reductase inhibitors have become widely used in recent years. Although generally well tolerated and effective, side effects have been reported in up to 4% of participants in controlled trials, including increases in serum levels of hepatic headache, and sleep disorders. With prolonged use, other side effects have been reported including depression, sensorimotor neuropathy and eczema. Alternative therapies are needed, especially for populations that cannot tolerate reductase inhibitors.

### SUMMARY OF THE INVENTION

It is an object of the present invention to provide active site inhibitors of cholesterol esterase for prevention of the hydrolysis of the cholesterol ester. By inhibiting cholesterol esterase the inhibitors of the present invention provide a new approach to the treatment of hypercholesterolemia through limiting the bioavailability of dietary cholesterol.

In one embodiment, the present invention provides a compound comprising: 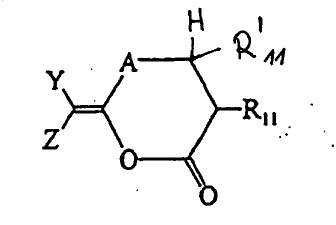 wherein
A = ―(CH₂)ₚ― where p = 0 or 1;
Y = H or C₁₋₈alkyl when Z = Cl, Br, or I and Y = Cl, Br, or I when Z = H or C₁₋₈ alkyl; and
R₁₁ is a member of the group consisting of: 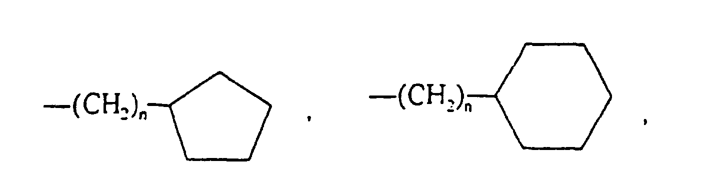 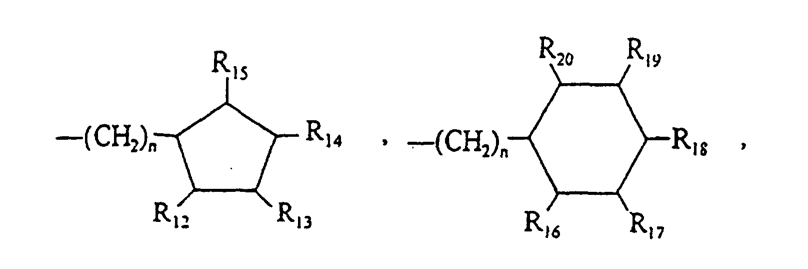 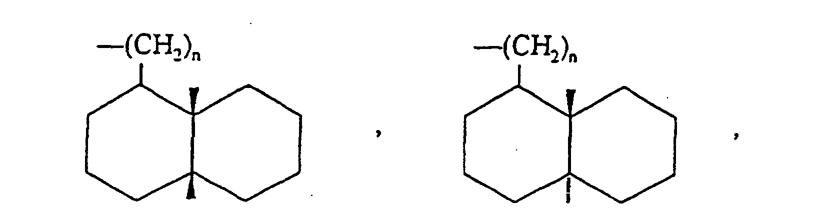 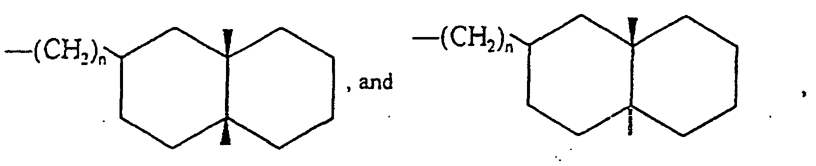 and -CHR₂₁-CHR₂₂-CHR₂₃-CH₂-,
wherein n = 0 to 8; and
R₁₂, R₁₃, R₁₄, R₁₅, R₁₆, R₁₇, R₁₈, R₁₉, R₂₀, R₂₁, R₂₂ and R₂₃ = H, C₁₋₈ alkyl, C₃₋₈ cycloalkyl, C₂₋₈ alkenyl, or C₂₋₈ alkynyl, and R'₁₁ = H or is a binding site for the CH₂-moiety of R₁₁ when R₁₁ = -CHR₂₁-CHR₂₂-CHR₂₃-CH₂-.

### BRIEF DESCRIPTION OF THE DRAWINGS

The invention will be described in conjunction with the accompanying drawings, in which:
Figure 1 illustrates one method for forming compounds of the present invention;
Figure 2 illustrates a second method for forming compounds of the present invention;
Figure 3 illustrates a third method for forming compounds of the present invention;
Figure 4 illustrates a fourth method for forming compounds of the present invention;
Figure 5 illustrates a fifth method for forming compounds of the present invention; and
Figure 6 is a graph showing the inhibition of cholesterol esterase by a compound of the present invention.

### DETAILED DESCRIPTION OF THE PREFERRED EMBODIMENTS

### Definitions

The term "C₁₋₈ alkyl" refers to a straight or branched chain alkyl moiety having one ot eight carbon atoms including for example, methyl, ethyl, propyl, isopropyl, butyl, sec-butyl, isobutyl, pentyl, dimethyl-propyl, hexyl, and octyl, and cognate terms (such as "C₁₋₈ alkoxy") are to be construed accordingly. Similarly, the term "C₁₋₅ alkyl" refers to a straight or branched chain alkyl moiety having one to five carbon atoms (such as methyl or ethyl).

The term "C₁₋₈cycloalkyl" refers to a saturated alicyclic moiety having from 3 to 8 carbon atoms arranged in a ring and includes, for example, cyclopropyl, cyclobutyl, cyclopentyl, and cyclooctyl.

The term "C₂₋₈ alkenyl" refers to a straight or branched chain alkyl moiety having one to eight carbon atoms and having in addition at least one double bond of either E or Z stereochemistry where applicable. This term would include, for example, vinyl, 1-propenyl, 1- and 2-butenyl and 2-methyl-2-propenyl.

The term "C₂₋₈ alkynyl" refers to a straight or branched chain alkyl moiety having one to eight carbon atoms and having in addition at least one triple bond. This term would include, for example, propargyl, and 1- and 2-butynyl.

For the purposes of the present invention, including the accompanying drawing figures, the possible values for the radicals R₁, R₂, R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, and R₂₃ are set forth above in the summary of the invention section.

For the purposes of the present invention, including the accompanying drawing figures, the letter "X" represents any one of the halide radicals Cl, Br and I and "X₂" represents any one of Cl₂, Br₂ and I₂.

### Description

Artherosclerotic heart disease is correlated to serum cholesterol levels. Therefore, a treatment which decreases the amount of dietary cholesterol absorbed from the intestine should also reduce a person's susceptibility to atherosclerotic heart disease.

The compounds of the present invention act as cholesterol esterase (CE) inhibitors, that is they prevent CE from hydrolyzing dietary cholesterol esters in the intestines. The compounds of the present invention have structures which allow them to enter the active site of CE, a serine esterase, and bind covalently to certain residues. This results in a permanent inactivation of the enzyme. The compounds of the present invention combine structural features of known good inhibitors of serine esterases and the structural features of a known potent irreversible inhibitor of CE.

Dietary cholesterol is comprised of free and esterified cholesterol, the ratio depending upon the dietary source. In diets rich in meats, up to 75% of cholesterol is esterified. Hydrolysis of cholesterol ester in the lumen of the small intestine is catalyzed by cholesterol esterase (CE) which liberates free cholesterol. Free cholesterol mixes with cholesterol contained in bile secretions to form the pool of cholesterol which is capable of being absorbed. Due to the low solubility of cholesterol, solubilization of cholesterol by bile salts into micelles is essential. In addition, transport proteins are required to deliver cholesterol from micelles to the enterocytes for absorption. CE provides both the hydrolytic activity for hydrolysis of cholesterol ester and the transport function for delivery of cholesterol from micelles to enterocytes. Esterification of cholesterol within the enterocyte also utilizes CE which can catalyze the reverse reaction under certain conditions. CE within the enterocyte is immunochemically related to pancreatic CE. Given the essential role of CE in hydrolysis of cholesterol esters and in cholesterol absorption, the present invention's active site inhibitors of CE are effective in reducing the bioavailabilty of dietary cholesterol.

The catalytic mechanism of pancreatic CE resembles that of serine proteases such as trypsin and serine esterases such as acetylcholine esterase. Sequence comparisons indicate that S194, H435 and D79 comprise the active site triad of serine, histidine and aspartic acid in pancreatic CE, corresponding to S195, H57 and D102 in trypsin. The importance of these residues in CE has been demonstrated by site directed mutagenesis studies by DiPersio *et al*. in "Site-directed mutagenesis of an essential histidine residue in pancreatic cholesterol esterase," *J*. *Biol*. *Chem*., 266, 4033-4036 (1991).

The feasibility of using a active site inhibitor to reduce cholesterol absorption has been reported by Bailey *et al* in. "Inhibition of dietary cholesterol ester absorption by 3-BCP, a suicide inhibitor of cholesterol esterase," *Biochem*. *Soc. Trans.,* 23, 408S (1995). Intragastric administration of a single dose of 3-benzyl-6-chloropyrone to rats simultaneous with feeding of cholesterol ester resulted in a 60% drop in cholesterol absorption, which resulted from a 63% inactivation of lumenal CE activity. In *vitro* CE was inactivated by this pyrone with a half life of 100 seconds. 3-Benzyl-6-chloropyrone is a prototype haloenol lactone that has been developed as a suicide inhibitor of chymotrypsin, although it is not highly selective as described in Daniels *et al*. in "Haloenol lactones. Potent enzyme-activated irreversible inhibitors for α-chymotrypsin," *J*. *Biol*. *Chem*., 250, 15046-15053 (1983) (Suicide inhibitors, also called suicide substrates, require catalytic activation to a form that irreversibly modifies the enzyme.). The compounds of the present invention improve on existing haloeonol lactone suicide inhibitors by being selective for CE.

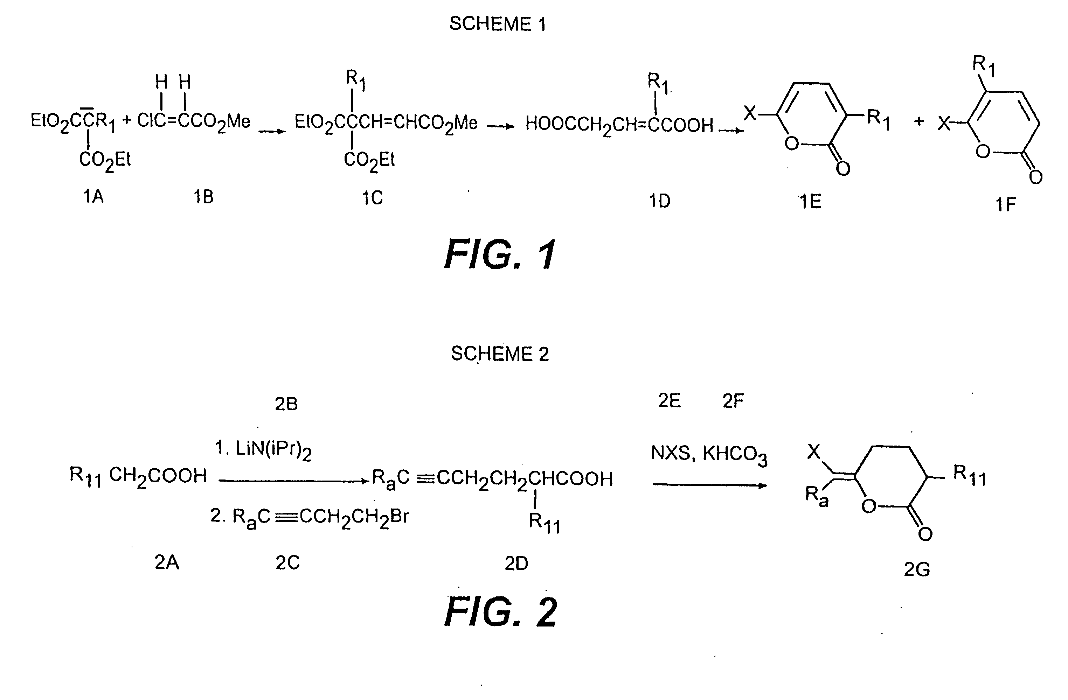

One method for synthesizing compounds of the present invention is Scheme 1 illustrated in Figure 1. An appropriately substituted diethylmalonate sodium salt (1A) is heated with a *cis*-2-chloroacrylate (1B) to give a triester compound (1C). Saponification and decarboxylation of the triester compound (1C) produces a substituted glutaconic acid (1D) which is often present as a mixture of the E and Z isomers. Cyclization is then performed using an acetyl halide cyclization agent (such as acetyl chloride or acetyl bromide) to give the 3-substituted 6-halopyrone (1E) and 5-substituted 6-halopyrone (1F). The 3-substituted 6-halopyrone of the invention (1E) is then separated form the other isomer and purified using conventional column chromatography techniques. The method of scheme 1 is similar to a procedure described by Boulanger and Katzenellenbogen in "Structure Activity Study of 6-Substituted 2-Pyranones as Inactivators of α-chymotrypsin" in *J*. *Med Chem*., 29, 1159-1163 (1986).

A second method for synthesizing compounds of the present invention is Scheme 2 illustrated in Figure 2. Cyclohexylacetic acid (2A) is reacted with two moles of lithium diisopropyl amide (2B) followed by alkylation with a 4-alkyl-1-bromo-3-butyne (2C) to produce an acetylenic acid intermediate (2D) (Where Rₐ = C₁₋₈ alkyl). Halolactone compounds of the present invention (2G) are then produced by halolactonization of the acetylenic acid intermediate (2D) with an N-halosuccinimide (NXS; 2E) and potassium bicarbonate (2F). Depending on the desired halide substituent (X) in the halolactone, the N-halosuccinimide (2E) can be N-bromosuccinimide, N-chlorosuccinimide or N-iodosuccinimide.

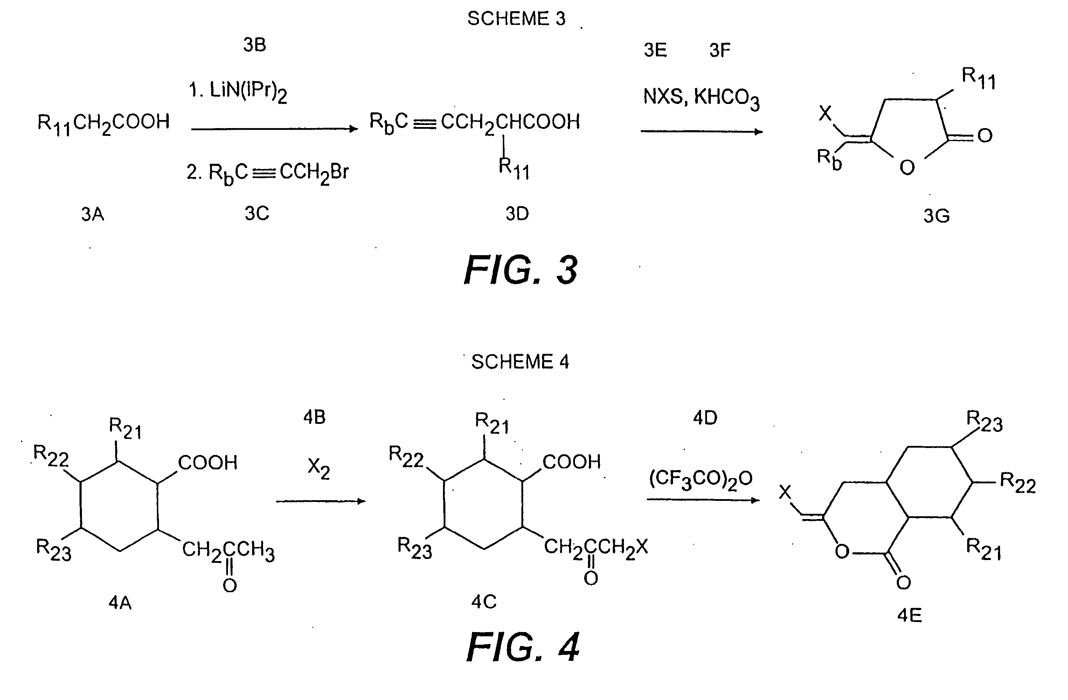

A third method for synthesizing compounds of the present invention is Scheme 3 illustrated in Figure 3. Cyclohexylacetic acid (3A) is reacted with two moles of lithium diisopropyl amide (3B) followed by alkylation with a 3-alkyl-1-bromo-2-propyne (3C) to produce an acetylenic acid intermediate (3D) (Where R_{b} = C₁₋₈ alkyl). Halolactone compounds of the present invention (3G) are then produced by halolactonization of the acetylenic acid intermediate (3D) with an N-halosuccinimide (NXS; 3E) and potassium bicarbonate (3F). Depending on the desired halide substituent (X) in the halolactone, the N-halosuccinimide (3E) can be N-bromosuccinimide, N-chlorosuccinimide or N-iodosuccinimide.

A fourth method for synthesizing compounds of the present invention is Scheme 4 illustrated in Figure 4. 2-(2-Oxopropyl)cyclohexanecarboxylic acid (4A) is reacted with a halogen X₂ (48) to produce a halo keto acid intermediate (4C). Depending on the desired halide substituent in the halolactone (4E), X₂ can be chlorine, bromine or iodine. Pyridinium tribromide can be used as the source of bromine. Halolactone compounds of the present invention (4E) are then produced by halolactonization of the intermediate (4C) with trifluoroacetic acid anhydride (4D).

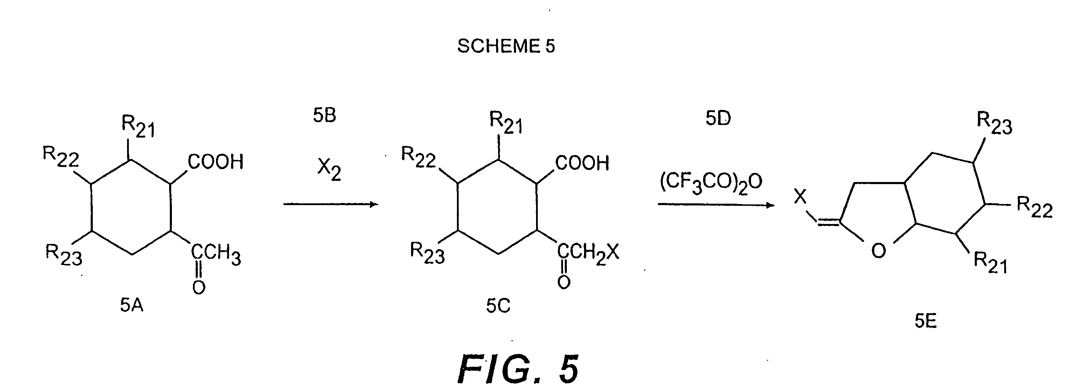

A fifth method for synthesizing compounds of the present invention is Scheme 5 illustrated in Figure 5. 2-Acetylcyclohexanecarboxylic acid (5A) is reacted with a halogen X₂ (5B) to produce a halo keto acid intermediate (5C). Depending on the desired halide substituent in the halolactone (5E), X₂ can be chlorine, bromine or iodine. Pyridinium tribromide can be used as the source of bromine. Halolactone compounds of the present invention (5E) are then produced by halolactonization of the intermediate (5C) with trifluoroacetic acid anhydride (5D).

The synthesis methods of scheme 2 and scheme 3 stereospecifically produce the E isomers 2G and 3G. The Z isomers of these compounds can be produced using a method similar to that described in Dai and Katzenellenbogen, "Stereoselective Z- and E- Bromo Enol Lactonization of Alkynoic Acids" in *J*. *Org. Chem*., 56, 6893-6896 (1991). For example, treating the silver salt of compound 2D with a halogen X in acetonitrile can be used to provide the following Z isomer compound of the present invention 2H: 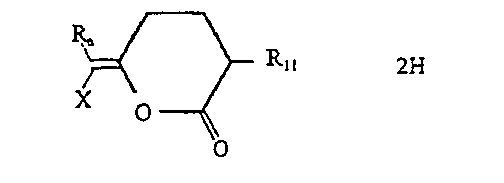 Similarly, treating the silver salts of compounds 3D, 4D, and 5D can be used to produce the following Z isomer compounds of the present invention 3H, 4H and 5H, respectively:

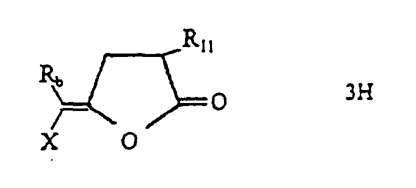

### EXAMPLE 1 (Reference)

A 3-substituted 6-chloropyrone of the invention was prepared according to Scheme 1,
wherein X = Cl, R₁ = 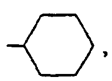 and acetyl chloride was used as a cyclization agent. NMR analysis of this compound provided the following results: H'NMR (CDCl₃): 7.02(d, 1H), 6.16 (d, 1H), 2.59 (m, 1H), 1.90-1.14 (m, 10H).

### EXAMPLE 2 (Reference)

A 3-substituted 6-chloropyrone of the invention was prepared according to Scheme 1, wherein X = Cl, R, = 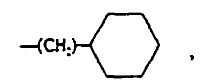 and acetyl chloride was used as a cyclization agent. NMR analysis of this compound provided the following results: H'NMR (CDCl₃):7.05 (d, 1H), 6.16 (d, 1H), 2.45 (d, 2H), 1.80-0.81 (m, 11H).

### EXAMPLE 3 (Reference)

A 3-substituted 6-chloropyrone of the invention was prepared according to Scheme 1, wherein X = Cl, R₁ = 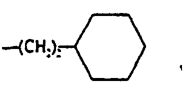 and acetyl chloride was used as a cyclization agent. NMR analysis of this compound provided the following results: H'NMR (CDCl₃):7.02(d, 1H), 6.11 (d, 1H), 2.39 (t, 2H), 1.72-0.85 (m, 13H).

### EXAMPLE 4 (Reference)

A 3-substituted 6-chloropyrone of the invention was prepared according to Scheme 1, wherein X = Cl, R₁ = 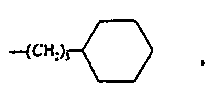 and acetyl chloride was used as a cyclization agent. NMR analysis of this compound provided the following results: H'NMR (CDCl₃):7.05 (d, 1H), 6.15 (d, 1H), 2.40 (t, 2H), 1.71-0.80 (m, 15H).

### EXAMPLE 5 (Reference)

A 3-substituted 6-chloropyrone of the invention was prepared according to Scheme 1, wherein X = Cl, R₁ = 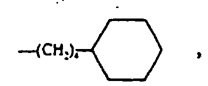 and acetyl chloride was used as a cyclization agent.

### EXAMPLE 6 (Reference)

A 3-substituted 6-chloropyrone of the invention was prepared according to Scheme 1, wherein X = Cl, R₁ = 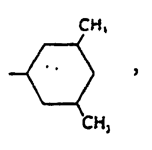 and acetyl chloride was used as a cyclization agent.

### EXAMPLE 7 (Reference)

A 3-substituted 6-chloropyrone of the invention was prepared according to Scheme 1, wherein X = Cl, R₁ = 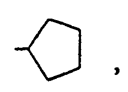 and acetyl chloride was used as a cyclization agent. NMR analysis of this compound provided the following results: H'NMR (CDCl₃):7.09(d,1H), 6.16(d,1H), 2.95 (pent, 1H), 2.00-1.46 (m8H).

### EXAMPLE 8 (Reference)

A 3-substituted 6-chloropyrone of the invention was prepared according to Scheme 1, wherein X = Cl, R₁ = 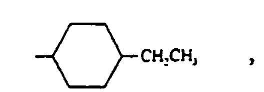 and acetyl chloride was used as a cyclization agent.

### EXAMPLE 9

A compound of the present invention is prepared according to Scheme 2, wherein X =Br, R₁ = C₆H₁₁, and n-bromosuccinimide is used as a cyclization agent.

### EXAMPLE 10

A compound of the present invention prepared according to Scheme 2, wherein X = Br, R₁ = C₇H₁₃, and n-bromosuccinimide is used as a cyclization agent.

### EXAMPLE 11

A compound of the present invention is prepared according to Scheme 2, wherein X = Br, R₁ = C₈H₁₅, and n-bromosuccinimide is used as a cyclization agent.

### EXAMPLE 12

A compound of the present invention is prepared according to Scheme 3, wherein X =Br, R₁ = C₆H₁₁, and n-bromosuccinimide is used as a cyclization agent.

### EXAMPLE 13

A compound of the present invention prepared according to Scheme 3, wherein X = Br, R₁ = C₇H₁₃, and n-bromosuccinimide is used as a cyclization agent.

### EXAMPLE 14

A compound of the present invention is prepared according to Scheme 3, wherein X = Br, R₁ = C₈H₁₅, and n-bromosuccinimide is used as a cyclization agent.

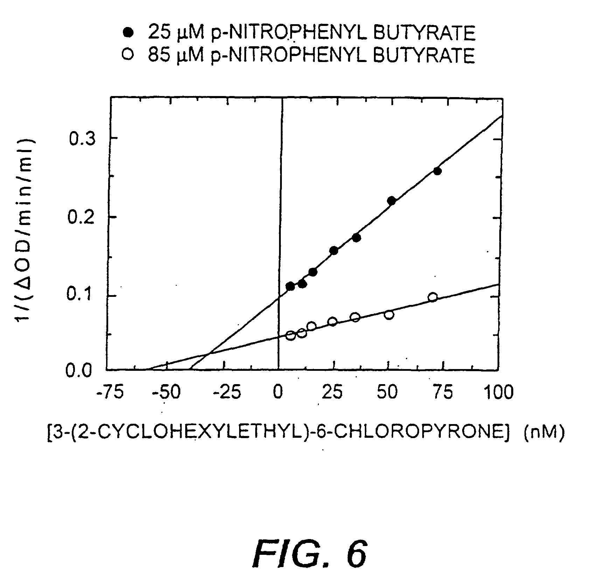

Kinetic inhibition studies of the compounds of Examples 1, 2, 3 and 4 revealed that all four of the compounds are potent inhibitors of CE. Representative data is shown in Figure 6 for inhibition of CE by 3-(2-cyclohexylethyl)-6-chloropyrone, the compound of Example 3. The data were analyzed by Dixon plots. As shown in Figure 6, the plot of I/V v [I] at two different fixed concentrations of the substrate *p*-nitrophenylbutyrate intersect above the x-axis, indicative of competitive inhibition. The calculated Kᵢ = 30ηM. However, a value of 30ηM corresponds to one half of the concentration of CE used in this assay, suggesting that the actual Kᵢ << 30ηM.

In inhibition studies with high affinity inhibitors under conditions where the concentration of enzyme used in the assay exceeds the concentration of the inhibitor, the Kᵢ obtained is an apparent Kᵢ corresponding to [E]/2. Therefore, clearly, the compounds of the present invention are potent inhibitors of CE.

The compounds of the present invention are also highly selective for CE, unlike 3-substituted 6-chloropyrones such as 3-benzyl-6-chloropyrone where the 3-substituent includes an aromatic ring. For example, the compound of Example 3 has been found to inhibit chymotrypsin with Kᵢ = 50µM compared to Kᵢ << 30ηM for inhibition of CE. Thus, the compounds of the present invention which have a saturated ring as part of the 3-substituent provide tremendous selectivity when compared to compounds where the 3-substituent includes an aromatic ring.

## Claims

1. A compound comprising: 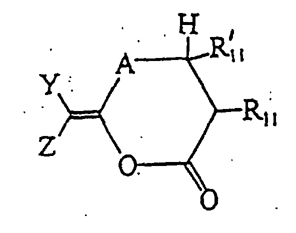 wherein A = -(CH₂)ₚ- and wherein p = 0 or 1;
Y = H or C₁₋₈ alkyl when Z = Cl, Br or I and Y = Cl, Br, or I when Z = H or C₁₋₈ alkyl;
R₁₁ is a member of the group consisting of: 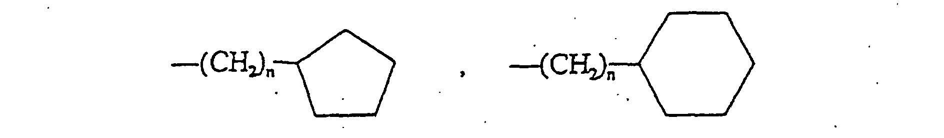 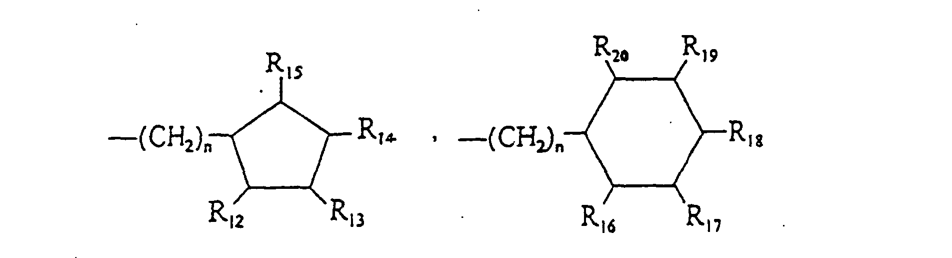 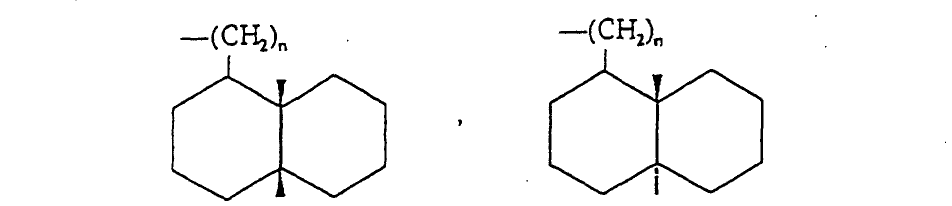 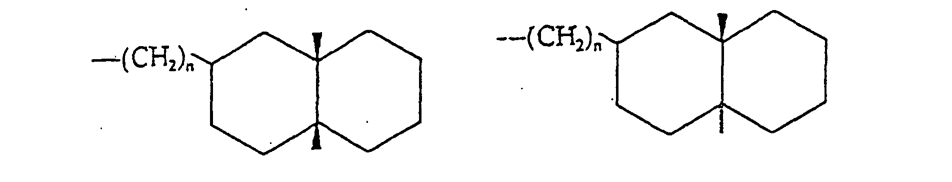 and - CHR₂₁ - CHR₂₂ - CHR₂₃ - CH₂-,
wherein n = 0 to 8;
R₁₂, R₁₃, R₁₄, R₁₅, R₁₆, R₁₇, R₁₈, R₁₉, R₂₀, R₂₁, R₂₂ and R₂₃ = H, C₁₋₈ alkyl, C₃₋₈ cycloalkyl, C₂₋₈ alkenyl, or C₂₋₈ alkynyl, and
R'₁₁ = H or is a binding site for the CH₂-moiety of R₁₁ when R₁₁ = - CHR₂₁ -CHR₂₂-CHR₂₃-CH₂-.

2. The compound of claim 1, wherein p = 0.

3. The compound of claim 1 wherein p = 1.

4. The compound of any one of claims 1 to 3, wherein Y = H or C₁₋₈ alkyl and Z = Cl, Br or I.

5. The compound of any one of claims 1 - 3, wherein Y = Cl, Br or I and Z = H or C₁₋₈ alkyl.

6. The compound of any one of claims 1 to 5 having the formula 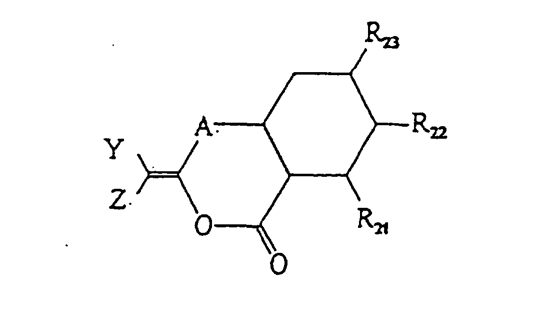 wherein A, Y, Z, and R₂₁, R₂₂, R₂₃ have the meanings claimed in the preceding claims.

## Patentansprüche

1. Verbindung aufweisend: 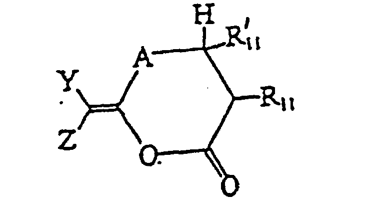 in der A = -(CH₂)ₚ- und in der p = 0 oder 1;
Y = H oder C₁₋₈ -Alkyl wenn Z = Cl, Br oder I, und Y = Cl, Br oder I wenn Z = H oder C₁₋₈-Alkyl;
R₁₁ ein Mitglied der Gruppe ist, die besteht aus: 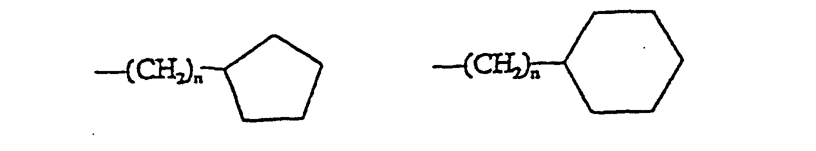 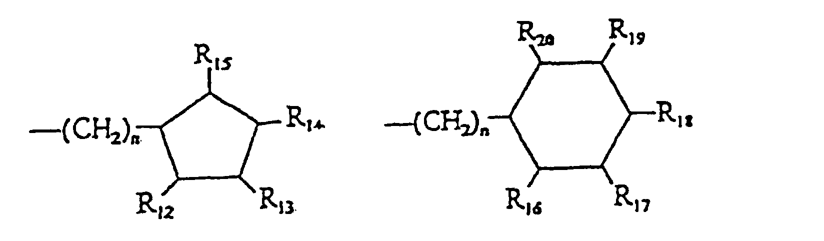 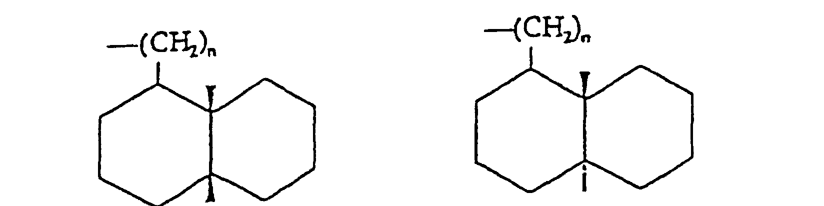 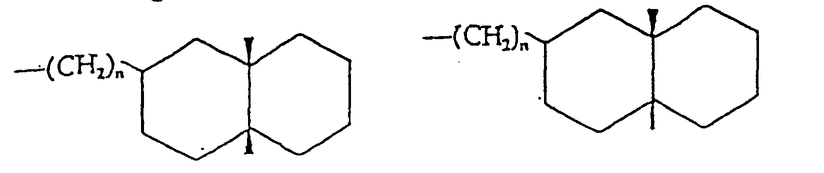 und -CHR₂₁ -CHR₂₂ -CHR₂₃ -CH₂-,
worin n = 0 bis 8;
R₁₂, R₁₃, R₁₄, R₁₅, R₁₆, R₁₇, R₁₈, R₁₉, R₂₀, R₂₁, R₂₂ und R₂₃ = H, C₁₋₈-Alkyl, C₃₋₈-Cycloalkyl, C₂₋₈ -Alkenyl, oder C₂₋₈-Alkinyl, und
R'₁₁ = H oder eine Bindungsstelle für die CH₂ -Gruppe von R₁₁ wenn R₁₁ = -CHR₂₁ -CHR₂₂ -CHR₂₃ -CH₂-.

2. Verbindung nach Anspruch 1, in der p = 0.

3. Verbindung nach Anspruch 1, in der p = 1.

4. Verbindung nach einem der Ansprüche 1 bis 3, in der Y = H oder C₁₋₈ -Alkyl und Z = Cl, Br oder I.

5. Verbindung nach einem der Ansprüche 1 bis 3, in der Y = Cl, Br oder I und Z = H oder C₁₋₈-Alkyl.

6. Verbindung nach einem der Ansprüche 1 bis 5 mit der Formel: 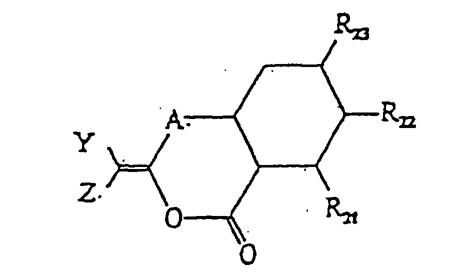 in der A, Y, Z und R₂₁, R₂₂, R₂₃ die in den vorangehenden Ansprüchen beanspruchten Bedeutungen haben.

## Revendications

1. Composé comprenant : 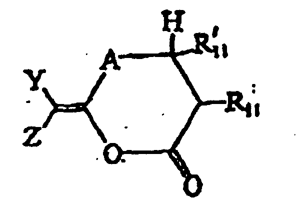 dans lequel A = -(CH₂)ₚ- et dans lequel p = 0 ou 1 ;
Y = H ou alkyle en C₁₋₈ quand Z = Cl, Br ou I, et Y = Cl, Br ou I quand Z = H ou alkyle en C₁₋₈ ;
R₁₁ est un élément du groupe constitué de : 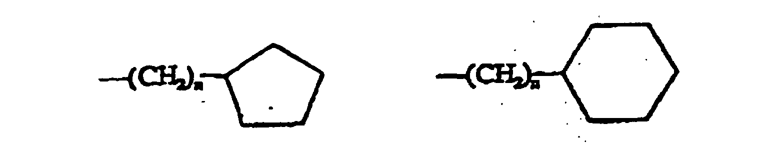 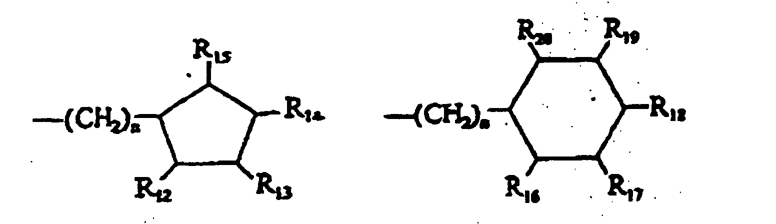 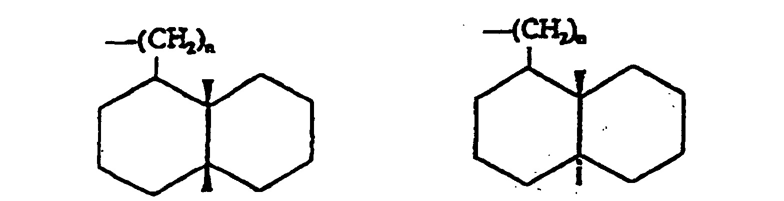 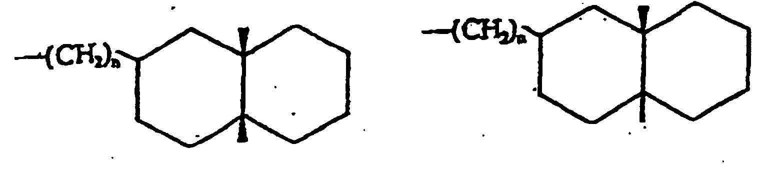 et -CHR₂₁-CHR₂₂-CHR₂₃-CH₂-,
où n = 0 à 8 ;
R₁₂, R₁₃, R₁₄, R₁₅, R₁₆, R₁₇, R₁₈, R₁₉, R₂₀, R₂₁, R₂₂ et R₂₃ = H, alkyle en C₁₋₈, cycloalkyle en C₃₋₈, alcényle en C₂₋₈ ou alkynyle en C₂₋₈, et R'₁₁ = H ou est un site de liaison pour le fragment CH₂ de R₁₁ quand R₁₁ = -CHR₂₁-CHR₂₂-CHR₂₃-CH₂-.

2. Composé selon la revendication 1, dans lequel p = 0.

3. Composé selon la revendication 1, dans lequel p = 1.

4. Composé selon l'une quelconque des revendications 1 à 3, dans lequel Y = H ou alkyle en C₁₋₈ et Z = Cl, Br ou I.

5. Composé selon l'une quelconque des revendications 1 à 3, dans lequel Y = Cl, Br ou I et Z = H ou alkyle en C₁₋₈.

6. Composé selon l'une quelconque des revendications 1 à 5, ayant la formule 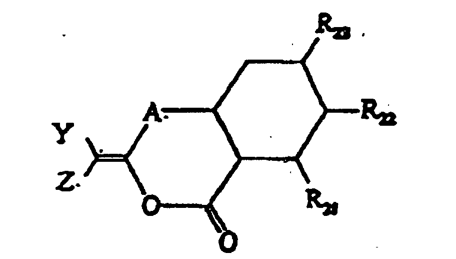 dans laquelle A, Y, Z et R₂₁, R₂₂, R₂₃ ont les significations revendiquées dans les revendications précédentes.
